# EUROPEAN PATENT SPECIFICATION

(11) **EP 1 919 508 B1**
(45) Date of publication and mention of the grant of the patent: **25.02.2015**
(21) Application number: 06765236.2
(22) Date of filing: 04.08.2006
(51) Int. Cl.: A61K 47/48

(54) **ARTIFICIAL PLATELETS**
KÜNSTLICHE BLUTPLÄTTCHEN
PLAQUETTES ARTIFICIELLES

(30) Priority: 04.08.2005 GB 0516091
(43) Date of publication of application: 14.05.2008
(60) Divisional application: 10015813.8
(73) Proprietor: HAEMOSTATIX LIMITED, Nottingham NG1 1GF (GB)
(72) Inventor: GOODALL, Alison, Pennyfoot Street Nottingham, NG1 1GF (GB); MIDDLETON, Sarah, Margaret, Pennyfoot Street Nottingham, NG1 1GF (GB); WALKER, Greg, Francis, Pennyfoot Street Nottingham, NG1 1GF (GB)
(74) Representative: Thornton, Neil
(86) International application number: PCT/GB2006/002934
(87) International publication number: WO 2007/015107

(56) References cited:
- EP-A2- 1 004 322
- WO-A-00/35487
- WO-A-98/17319
- WO-A-99/24146
- WO-A-2005/035002
- WO-A1-99/42146
- WO-A2-2005/123760
- US-A- 6 015 561
- US-A1- 2003 143 158
- US-B1- 6 184 205
- Yin C. Lin ET AL: "A Latent Inhibitor of Fibrin Polymerization with Ancillary Anticoagulant Activity", Thrombosis Research, vol. 97, no. 5, 1 March 2000 (2000-03-01), pages 375-378, XP055017399, ISSN: 0049-3848, DOI: 10.1016/S0049-3848(99)00170-X

## Description

This invention relates to agents suitable for use as artificial platelets. The agents may be used to treat patients with deficiencies in their own platelets, such as hereditary or acquired defects of platelet numbers (thrombocytopenia) or function (thrombasthenia).

Platelet transfusion is currently the only effective treatment for acute bleeding and the prevention of bleeding in patients with disorders of platelet production and/or function. However, the shelf-life of platelet concentrates used for transfusion is only five days. There is also a risk of infection associated with platelet transfusion, and a risk that the patient may have an allergic reaction to platelet concentrates.

In view of the problems associated with platelet concentrates and platelet transfusion, attempts have been made to develop artificial platelets. It is important that artificial platelets aggregate with each other and endogenous platelets at the site of blood vessel damage where platelets are activated, but that minimal aggregation occurs elsewhere. When platelets are activated, a conformational change in a platelet membrane glycoprotein, GPIIb-IIIa, allows it to bind plasma fibrinogen and recruit further platelets into a growing thrombus.

WO 2005/035002 describes production of two artificial platelet products. Product 1 is a peptide of sequence NH₂-GPRPGGGGGGC (SEQ ID NO: 1) cross-linked through the terminal cysteine to a human albumin microsphere. Fibrinogen is bound to the peptide. Product 2 is identical to Product 1 except that fibrinogen is not bound to the peptide. When administered, Product 2 binds endogenous fibrinogen *via* the peptide. The peptide is capable of binding to fibrinogen such that fibrinogen is maintained in, or close to, its native conformation. This allows preferential interaction with the activated form of the GPIIb-IIIa receptor. The bound fibrinogen can also interact with thrombin, which cleaves fibrinogen, allowing cross-linking of the microspheres through fibrin-fibrin bridges.

It is believed that Product 2 will have an enhanced safety profile compared with Product 1 because Product 2 binds to the patient's endogenous fibrinogen. This reduces the risk of infection and allergic reaction when Product 2 is administered.

It is desired to provide further improved artificial platelets. In particular, there is a need to provide artificial platelets in which the risk of thrombus formation away from a site of blood vessel damage is further minimised.

There is provided an agent for use as an artificial platelet, the agent comprising a fibrinogen binding precursor bound to an insoluble carrier, wherein the fibrinogen binding precursor can be converted by a wound site specific agent to a fibrinogen binding component bound to the carrier, the fibrinogen binding component having increased ability to bind fibrinogen compared to the fibrinogen binding precursor, and the fibrinogen binding precursor not being fibrinogen.

In particular, according to the invention, there is provided an agent as defined in the claims below.

Once the fibrinogen binding precursor has been converted to the fibrinogen binding component at the site of a wound, the fibrinogen binding component is able to bind fibrinogen such that the bound fibrinogen can bind to platelets, thereby causing platelet aggregation. Because a wound site specific agent is required to convert the fibrinogen binding precursor, platelet aggregation away from the site of a wound is minimised. It is believed that agents of the invention will have reduced thrombogenicity away from the wound compared to known artificial platelets and, therefore, enhanced safety.

A further advantage of agents of the invention is that it is not essential that the fibrinogen binding component binds to fibrinogen in such a way as to maintain fibrinogen in, or close to, its native confirmation so that the bound fibrinogen preferentially interacts with activated platelets. This is because the agents are only activated at the site of a wound. Consequently, the choice of fibrinogen binding component is not as critical as for Products 1 and 2 described in WO 2005/035002. Nonetheless, it is preferred that the fibrinogen binding component binds to fibrinogen such that the bound fibrinogen interacts preferentially with activated platelets. This further minimises the risk that the agents will cause thrombus formation away from a wound site.

Once fibrinogen is bound to the fibrinogen binding component, it should be able to be cleaved by thrombin to allow cross-linking of the insoluble carriers through fibrin-fibrin bridges.

Preferably the fibrinogen binding component binds to fibrinogen with a dissociation constant (K_{D}) of between 10⁻⁹ to 10⁻⁶ M.

The dissociation constant can be measured at equilibrium. For example, radio labelled fibrinogen of known concentration can be incubated with microspheres to which the fibrinogen binding component has been cross-linked. Typically 5µM peptide is cross-linked to 1gm microspheres, or 15-40 µmoles of peptide is cross-linked to 1gm of microspheres. The peptide-linked microspheres are diluted to 0.5 mg/ml, and incubated in isotonic buffer at pH 7.4 (for example 0.01 M Hepes buffer containing 0.15M NaCl) with radio labelled fibrinogen at concentrations of between 0.05 and 0.5mg/ml for up to 1 hr at 20°C. The fibrinogen bound to the fibrinogen binding component on the microspheres can be separated from the free fibrinogen by centrifugation and the amount of free and bound fibrinogen measured. The dissociation constant can then be calculated by Scatchard analysis by plotting concentration of bound fibrinogen against the ratio of the concentrations of bound: free fibrinogen, where the slope of the curve represents K_{D}.

It will be appreciated that the lower the affinity of the fibrinogen binding precursor (and the insoluble carrier) for fibrinogen the better. This will minimise the risk of agents of the invention causing platelet aggregation unless converted by a wound site specific agent at the site of a wound.

Administration of an agent of the invention to a patient should not cause medically unacceptable levels of blood coagulation away from a wound site. Medically unacceptable levels of blood coagulation include deep vein thrombosis (DVT), pulmonary embolism (PE), transient cerebral or cardiovascular ischemia or stroke.

Preferably the dissociation constant of the fibrinogen binding precursor for fibrinogen is greater than 1x10⁻⁶M.

The fibrinogen binding precursor is a peptide.

The fibrinogen binding precursor comprises a fibrinogen binding peptide (i.e. the fibrinogen binding component) joined at its amino terminal end to a blocking component (a peptide) that blocks or inhibits (i.e. reduces) binding of fibrinogen to the fibrinogen binding peptide. Cleavage of the fibrinogen binding precursor by the wound site specific agent exposes the fibrinogen binding peptide bound to the insoluble carrier. In such embodiments, the blocking component blocks or inhibits the ability of the fibrinogen binding peptide to bind fibrinogen until cleavage occurs. The blocking component is a peptide of 1-30 amino acid residues in length.

The fibrinogen binding peptide may comprise the sequence of residues which becomes exposed on fibrinogen by the action of thombin, and which binds fibrinogen as the first step in the polymerisation reaction to produce fibrin. Thrombin cleaves peptides (releasing fibrinopeptides A and B) from the N terminals of the α and β chains of fibrinogen to expose the sequences NH₂-GPR-(SEQ ID NO: 14) and NH₂-GHR- (SEQ ID NO: 15) respectively. The fibrinogen binding peptide comprises the amino acid sequence NH₂-G(P,H)RX- (SEQ ID NO: 16) at its amino terminal end, where X is any amino acid, and (P,H) means that either proline or histidine is present at that position. Preferably the peptide comprises the sequence NH₂-GPRP- (SEQ ID NO: 17) at its amino terminal end.

The fibrinogen binding peptide is 4-30, preferably 4-10, amino acid residues in length.

The wound site specific agent is thrombin, a naturally occurring enzyme, generated at the site of the wound, which is capable of cleaving the fibrinogen binding precursor. The fibrinogen binding precursor comprises a cleavage site that is recognised specifically by the wound site specific agent, and which is located between the fibrinogen binding peptide and the blocking component. Other serine proteases, such as Factor Xa or FIXa, may be generated at the wound site. Thrombin is known to cleave peptide bonds carboxy-terminal to arginine residues, and commonly between an arginine and a glycine.

In preferred embodiments of the invention the fibrinogen binding precursor is a peptide which comprises the amino acid sequence NH₂-ZYXR/GPRP- (SEQ ID NO: 18) at its amino terminal end, where "/" represents a thrombin cleavage site, and X is any amino acid, but is preferably proline, Y is any amino acid, but is preferably aspartic acid or alanine, and Z is at least one amino acid that is preferably leucine or proline. Examples are: NH₂-LVPR/GPRP- (SEQ ID NO: 19), NH₂-ADPR/GPRP- (SEQ ID NO: 20), NH₂-LDPR/GPRP- (SEQ ID NO: 21), or NH₂-LVPR/GPRV- (SEQ ID NO: 22).

In preferred embodiments, an agent of the invention further comprises a binding site for the wound site specific agent. This is expected to enhance conversion of the fibrinogen binding precursor to the fibrinogen binding component at a wound site. The binding site may be on the insoluble carrier, bound to the fibrinogen binding precursor, or part of the fibrinogen binding precursor. In a preferred embodiment, the binding site is bound to the fibrinogen binding component such that cleavage of the fibrinogen binding precursor by the wound site specific agent releases the binding site to expose the fibrinogen binding component. For example, the fibrinogen binding precursor may be a peptide which comprises, in the carboxy terminal to amino terminal direction, a fibrinogen binding peptide immobilised to the insoluble carrier, a cleavage site for the wound site specific agent, and a peptide comprising a binding site for the wound site specific agent. Typically, the carboxy terminal end of the binding site peptide will need to be separated from the amino terminal end of the fibrinogen binding peptide by sequence that is recognised by the wound site specific agent when it cleaves the fibrinogen binding precursor peptide at the cleavage site. For example, where the amino terminal residue of the fibrinogen binding peptide is G, the sequence may be RXYZ (in the carboxy- to amino- direction) (SEQ ID NO: 23) as defined above. Optionally a spacer may also be present between the cleavage sequence recognised by the wound site specific agent and the binding site peptide. The spacer should be long enough to allow the wound site specific agent to be able to cleave the fibrinogen binding precursor when it is bound to the binding site peptide. Preferably the spacer is a peptide of 1-20 amino acid residues in length.

It is preferred that an agent of the invention further comprises a thrombin binding site. The thrombin binding site is preferably provided by a peptide to which thrombin can bind, preferably with an affinity of Kd.10⁻⁹ to 10⁻⁶. Preferably the peptide comprises a sequence to which an exocite I or II binding domain of thrombin can bind. Suitable examples of thrombin binding peptides are sequences derived from the exocite binding site of the PAR-1 receptor (such as WEDEEKNES (SEQ ID NO: 24)), fibrinogen (such as VRPEHPAETEYDSLYPEDDL (SEQ ID NO: 25)), or Factor VIII (such as EEEDWD (SEQ ID NO: 26) or EDSYED (SEQ ID NO: 27)). Alternatively, the thrombin binding site may be derived from the thrombin binding peptide hirudin (for example NGDFEEIPEEYL (SEQ ID NO: 28)). The thrombin binding peptide may be cross-linked as a separate peptide (by an appropriate linker, either a peptide or a non-peptide) to the insoluble carrier. Alternatively, the thrombin binding peptide may be part of a fibrinogen binding precursor peptide.

In preferred embodiments, an agent of the invention further comprises a wound site targeting component to promote localisation of the agent to a wound site. The wound site targeting component should bind selectively to a wound site (preferably with an affinity of Kd 10⁻⁹ to 10⁻⁶). Preferably the wound site targeting component is capable of binding to cell surface protein tissue factor. Blood clotting is triggered when cell surface protein tissue factor is exposed to blood following vascular injury. Once exposed, cell surface tissue factor binds the inert precursor of a serine protease Factor VII, or the enzymically active form VIa, with a very high affinity and specificity. Thus, the wound site targeting component may comprise Factor VII or a fragment or derivative thereof capable of binding cell surface protein tissue factor, or Factor VIIa or a fragment or derivative thereof capable of binding cell surface protein tissue factor.

The wound site targeting component may be immobilised to the insoluble carrier, bound to the fibrinogen binding precursor, or part of the fibrinogen binding precursor. In one embodiment the wound site targeting component and the fibrinogen binding precursor are cross-linked separately to the insoluble carrier. In another embodiment the wound site targeting component is bound to the fibrinogen binding component such that cleavage of the fibrinogen binding precursor by the wound site specific agent releases the wound site targeting component to expose the fibrinogen binding component. For example, the fibrinogen binding precursor may be a peptide which comprises, in the carboxy terminal to amino terminal direction, a fibrinogen binding peptide immobilised to the insoluble carrier, a cleavage site for the wound site specific agent, and a wound site targeting component. Typically, the carboxy terminal end of the wound site targeting component will need to be separated from the amino terminal end of the fibrinogen binding peptide by sequence that is recognised by the wound site specific agent when it cleaves the fibrinogen binding precursor peptide at the cleavage site. For example, where the amino terminal residue of the fibrinogen binding peptide is G, the sequence may be RXYZ (in the carboxy- to amino-direction) as defined above. Optionally a spacer may also be present between the cleavage sequence recognised by the wound site specific agent and the wound site targeting component. The spacer should be long enough to allow the wound site targeting component to be able to bind selectively to the wound site. Preferably the spacer is a peptide of 1-20 amino acid residues in length.

The insoluble carrier should be inert, biocompatible, and should not cause any medically unacceptable levels of blood coagulation when the agent is administered. The insoluble carrier should be small enough to allow transmission of the agent through the lung capillary bed. The ability of an agent to be transmitted through the lung capillary bed can be determined using the method of Perkins et al. 1997, The British Journal of Radiology, 70, 603.

The insoluble carrier may have a maximum dimension such that a minority, for example less than about 2% of the population by number, are in excess of 6µm as a maximum dimension, as measured by a particle counter, such as a Coulter Multizer II. A size of from 2 to 4 µm as a maximum dimension may be suitable, which is comparable to the size of human platelets.

The insoluble carrier may be a microparticle (including a solid, hollow, or porous microparticle), preferably a substantially spherical microparticle. The microparticle may be formed of any suitable substance, for example cross-linked protein. A suitable protein is albumin (serum-derived or recombinant, human or non human). Microparticles suitable for use as insoluble carriers in the present invention may be formed by spray drying human serum albumin using well known spray drying technology, for example as in WO 92/18164.

Alternatives to use of microparticles as carriers include liposomes, synthetic polymer particles (such as polylactic acid, polyglycolic acid and poly(lactic/glycolic) acid), or cell membrane fragments.

The fibrinogen binding precursor is covalently bound to the insoluble carrier. Examples of preferred covalent bonds are a disulphide bond, a thioether bond, or an amide bond. A suitable covalent bond can be formed when the fibrinogen binding precursor is a peptide which comprises a cysteine and the carrier comprises a thiol reactive group. This allows the peptide to be bound to the carrier by linking the -SH group of the cysteine to the thiol-reactive group on the carrier. Preferably a terminal cysteine group is incorporated in the fibrinogen binding precursor peptide to crosslink the peptide with a thiol-reactive group on the carrier. Alternatively, a covalent bond may be formed when the fibrinogen binding precursor is a peptide which comprises a maleimide group (preferably at its carboxy terminus, for example attached to a carboxy-terminal lysine of the peptide), and the carrier comprises a sulphydryl group. The peptide may then be bound to the carrier by reacting the maleimide group of the peptide with the sulphydryl group of the carrier.

Typically, a spacer will be required between the fibrinogen binding component and the insoluble carrier to ensure that the fibrinogen-binding activity of the fibrinogen binding component is not adversely affected by the insoluble carrier. For example, the surface of the insoluble carrier may sterically hinder binding of fibrinogen to the fibrinogen binding component unless this is a sufficient distance from the surface of the insoluble carrier.

The fibrinogen binding precursor is a peptide which comprises a fibrinogen binding peptide, and the precursor is bound to the insoluble carrier by a terminal amino acid residue. A spacer sequence is preferably present between the terminal amino acid residue and the fibrinogen binding peptide of the precursor. The spacer sequence may, for example, be from 1-20, preferably 5-20, amino acid residues in length. The spacer sequence GGGGGG (SEQ ID NO: 29) or GGGGG (SEQ ID NO: 30) is preferred.

Any number of fibrinogen binding precursors may be bound to the insoluble carrier. A platelet typically has 50,000-100,000 GPIIb-IIIa surface proteins. A similar number of fibrinogen binding precursors bound to the insoluble carrier may be appropriate, although around 1,000-10,000 is preferred.

According to the invention there is also provided a pharmaceutical composition comprising an agent of the invention, and a pharmaceutically acceptable carrier, excipient, or diluent.

The agent may be formulated in suspension in an isotonic buffer at physiological pH. A buffer containing mannitol, glucose, human albumin, or trahalose could be used. The agent may be lyophilised to produce a freeze dried dosage form which can be reconstituted immediately prior to use.

The agent is typically produced aseptically. Preferably the agent is additionally subjected to a terminal sterilisation treatment. An example of a suitable treatment is heating a liquid suspension at a suitable temperature, for example 60°C, for 10 hours, or steam sterilisation in an autoclave at 121°C for 15minutes. Alternatively, the agent can first be lyophilised and then heated to, for example, 160°C for at least 2hrs, or 80°C for 72 hours. Alternatively, the heat treatment step could be replaced by gamma irradiation, for example by exposure to 25-35kGy using a cobalt⁶⁰ source.

There is also provided according to the invention an agent of the invention for use as a medicament.

There is further provided according to the invention use of an agent of the invention in the manufacture of a medicament for preventing, treating, or ameliorating thrombocytopenia or thrombasthenia.

Thrombocytopenia is diagnosed by counting blood cells. The normal platelet count is 150-400x10⁹/l. Below this range primary haemostasis is impaired and bleeding time prolonged. However, spontaneous life threatening bleeding will usually only occur when the platelet count drops under 10x10⁹/l. Methods of the invention may be used where the subject has a platelet count below 400x10⁹/l, preferably below 150x10⁹/l, more preferably below 10x10⁹/l.

The most common cause of thrombocytopenia is a failure in platelet production from the bone marrow, such as in blood cancers or following cytotoxic chemotherapy or radiotherapy.

Thrombocytopenia may result from conditions that cause increased platelet destruction. These include Immune thrombocytopenic purpura, disseminated intravascular coagulation, heparin-induced thrombocytopenia, other drug-induced thrombocytopenias, systemic lupus erythematosus, HIV-1-related thrombocytopenia, thrombotic thrombocytopenia purpura/haemolytic-uremic syndrome, common variable immunodeficiency, and post-transfusional purpura.

Thrombocytopenia may result from conditions that cause decreased platelet production. These include thrombocytopenia with absent radii (TAR) syndrome, amegakaryocytic thrombocytopenia, giant platelet syndromes (such as Bernard-Soulier syndrome, May-Hegglin anomaly, Fechtner syndrome, Sebastian syndrome, Epstein syndrome, Montreal platelet syndrome), and Wiskott-Aldrich syndrome.

Thrombocytopenia may result from conditions that cause sequestration (for example hypersplenism or Nasabach-Merritt syndrome) or increased platelet destruction and hemodilution (such as extracorporeal perfusion).

Thrombasthenia (acquired platelet function defects) may result from uremia, myeloproliferative disorders (such as essential thrombocythemia, polycythemia vera, chronic myeloid leukaemia, and agnogenicmyeloid metaplasia), acute leukaemias and myelodysplatic syndromes, dysproteinemias, extracorporeal perfusion, acquired von Willebrands disease, acquired storage pool deficiency, antiplatelet antibodies, liver disease.

Thrombasthenia (inherited platelet function defects) may result from platelet adhesion conditions (such as Bernard-Soulier syndrome), agonist receptor conditions (such as integrin α2β1 (collagen receptor) deficiency, P2Y₁₂ (ADP receptor) deficiency or thromboxane A₂ receptor deficiency), signalling pathway conditions (such as Gαq deficiency, phospholipase C-β2 deficiency, cyclooxygenase deficiency, thromboxane synthetase deficiency, lipoxygenase deficiency or defects in calcium mobilisation), secretion conditions (such as storage pool disease, Hermansky-Pudlak syndrome, Chediak-Higashi syndrome, Gray platelet syndrome, Quebec syndrome and Wiskott-Aldrich syndrome), aggregation conditions (such as Glanzmann thrombasthenia or congenital afibringenemia) and platelet-coagulant protein interaction conditions (such as Scott syndrome).

The agents may be used to prevent, treat, or ameliorate any of the above conditions.

The agents may also be used to treat patients who have sustained mechanical damage to their platelets, such as occurs during extra corporeal circulation in coronary bypass surgery and/or haemodialysis.

A suitable total dosage of the agent is expected to be in the range 0.1-5g protein, or 0.1-5g protein equivalents of protein-based carrier.

Also according to the invention there is provided a fibrinogen binding precursor for use as part of an agent of the invention. The fibrinogen binding precursor is a peptide comprising the amino acid sequence NH₂-ZYXR/GPRP-(SEQ ID NO: 18) at its amino terminal end, where "/" represents a thrombin cleavage site, and X is proline, Y is aspartic acid or alanine, and Z is leucine or proline. In a particularly preferred embodiment the fibrinogen binding precursor peptide comprises an amino acid sequence of any of SEQ ID NOs: 32-35, or 38-41.

There is also provided according to the invention a method of identifying a fibrinogen binding precursor for use as part of an agent of the invention, which comprises:
i) incubating a wound site specific agent with a labelled candidate fibrinogen binding precursor bound to an insoluble carrier under conditions that permit conversion of known fibrinogen binding precursors to fibrinogen binding components by the wound site specific agent;
ii) determining the amount of label on the insoluble carrier before and after incubation with the wound site specific agent; and
iii) identifying the candidate fibrinogen binding precursor as a fibrinogen binding precursor if the amount of label on the insoluble carrier after incubation with the wound site specific agent is less than the amount of label on the insoluble carrier before incubation with the wound site specific agent.

An example of such a method is described in Example 11 below.

The methods may further comprise determining binding of fibrinogen to the insoluble carrier before and after incubation with the wound site specific agent, and in step (iii) identifying the candidate fibrinogen binding precursor as a fibrinogen binding precursor if the amount of label on the insoluble carrier after incubation with the wound site specific agent is less than the amount of label on the insoluble carrier before incubation with the wound site specific agent, and the binding of fibrinogen to the insoluble carrier is increased after incubation with the wound site specific agent.

There is further provided according to the invention a method of identifying a fibrinogen binding precursor for use as part of an agent of the invention, which comprises:
i) contacting a wound site specific agent with a candidate fibrinogen binding precursor bound to an insoluble carrier under conditions that permit conversion of fibrinogen binding precursors to fibrinogen binding components by the wound site specific agent;
ii) determining whether the candidate fibrinogen binding precursor promotes clot formation, platelet aggregation, or aggregation of the insoluble carrier, after it has been contacted with the wound site specific agent; and
iii) identifying the candidate fibrinogen binding precursor as a fibrinogen binding precursor if clot formation is promoted.

Suitable methods for determining whether a candidate fibrinogen binding precursor promotes clot formation, platelet aggregation, or aggregation of the insoluble carrier, after contact with the wound site specific agent thrombin are described in Examples 7-10 below.

It will be appreciated that it is in general important that an agent of the invention is not degraded (or only slowly degraded) once administered (except for its conversion by a wound site specific agent at the site of a wound). Preferably the circulation time of an agent of the invention in the blood is greater than one hour, once administered to a subject who does not have any wound sites that would cause conversion of the agent. A suitable method for determining the stability of an agent of the invention *in vitro* is described in Example 12 below. Agents of the invention should be stable under the conditions of the test described in Example 12 for at least one hour.

Examples 1-4 of WO 2005/035002 describe methods of producing peptide linked microspheres, methods of assaying fibrinogen binding to peptide-linked microspheres, modified aggregation assays, *in vitro* activity assays, and *in vivo* evaluation models. It will be appreciated that corresponding methods may be used to identify, produce, and test agents of the present invention.

Preferred embodiments of the invention are now described, by way of example only, with reference to the accompanying drawings in which:
(Figure 1 shows schematically a first preferred embodiment of an agent of the invention;
Figure 2 shows schematically a second preferred embodiment of an agent of the invention;
Figure 3a shows schematically a third preferred embodiment of an agent of the invention; Figure 3b shows schematically a fourth preferred embodiment of an agent of the invention;
Figure 4 which shows the results of measurement of binding of fibrinogen to peptide-linked microspheres as described in Example 3 below;
Figure 5 shows the percentage of fibrinogen bound microspheres after incubation with various concentrations of thrombin. Microspheres without peptide modification at 0.7 mg/ml (▲) and microspheres modified with peptide C at 0.7 (● ), 5 ( **■**) and 10 mg/ml (▼);
Figure 6 shows an aggregation profile of blank or peptide C modified microparticles with fibrinogen in the absence (1 and 2) or presence of thrombin at 0.032 U/ml (3 and 4) respectively;
Figure 7 shows an aggregation profile of peptide C modified microparticles with plasma which had not been exposed to thrombin (1) and have been pre-incubated with thrombin and added to plasma to which thrombin is added after two minutes (2);
Figure 8 shows an aggregation profile of blank or peptide C modified microparticles with plasma. Peptide C (1) and blanks (2) microparticles without thrombin; Blanks (3) and peptide C (4) microspheres with the addition of thrombin at two minutes; and
Figure 9 shows platelet aggregation in thrombocytopenic blood activated by adenosine diphosphate (ADP) with and without microparticles that have been pre exposed to thrombin, using platelet aggegometry.

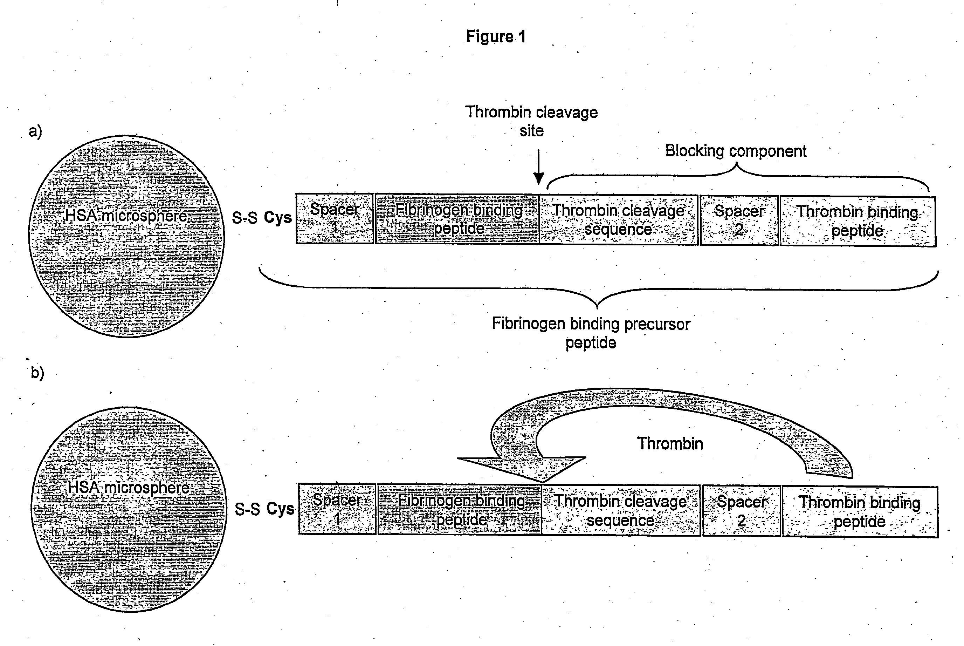

A first preferred embodiment of an agent of the invention is shown schematically in Figure 1 (a). The agent comprises a fibrinogen binding precursor peptide bound to a Human Serum Albumin (HSA) microsphere. The fibrinogen binding precursor peptide comprises (from its carboxy-terminal end to its amino terminal end): a carboxy-terminal cysteine residue which is cross-linked to the HSA microsphere; a first spacer sequence (Spacer 1); a fibrinogen binding peptide; a thrombin cleavage sequence (the box in the figure labelled "Thrombin cleavage sequence" represents the sequence which is amino terminal to the site at which thrombin is able to cleave the fibrinogen binding precursor peptide, i.e. the thrombin cleavage site); a second spacer sequence (Spacer 2); and a thrombin binding peptide.

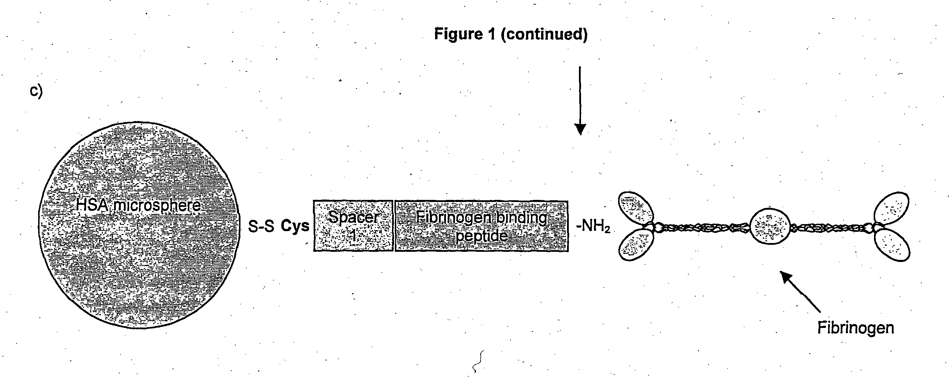

The portion of the fibrinogen binding precursor peptide which is on the amino-terminal side of the fibrinogen binding peptide (i.e. the Thrombin cleavage sequence-Spacer 2-Thrombin binding peptide portion) forms a blocking component. The blocking component blocks or inhibits binding of fibrinogen to the fibrinogen binding peptide. Thrombin generated at a wound site is able to bind to the thrombin binding peptide and cleave the fibrinogen binding precursor peptide at the amino terminal end of the fibrinogen binding peptide (as shown in Figure 1(b)). Cleavage of the fibrinogen binding precursor peptide releases the blocking component from the fibrinogen binding peptide, thereby exposing the fibrinogen binding peptide and allowing binding of fibrinogen to the fibrinogen binding peptide (Figure 1(c)).

The first spacer spaces the fibrinogen binding peptide from the HSA microsphere so that the microsphere does not interfere with binding of fibrinogen to the fibrinogen binding peptide once this has been exposed. The second spacer allows thrombin to be optimally orientated to cleave the thrombin cleavage site when it is bound to the thrombin binding peptide.

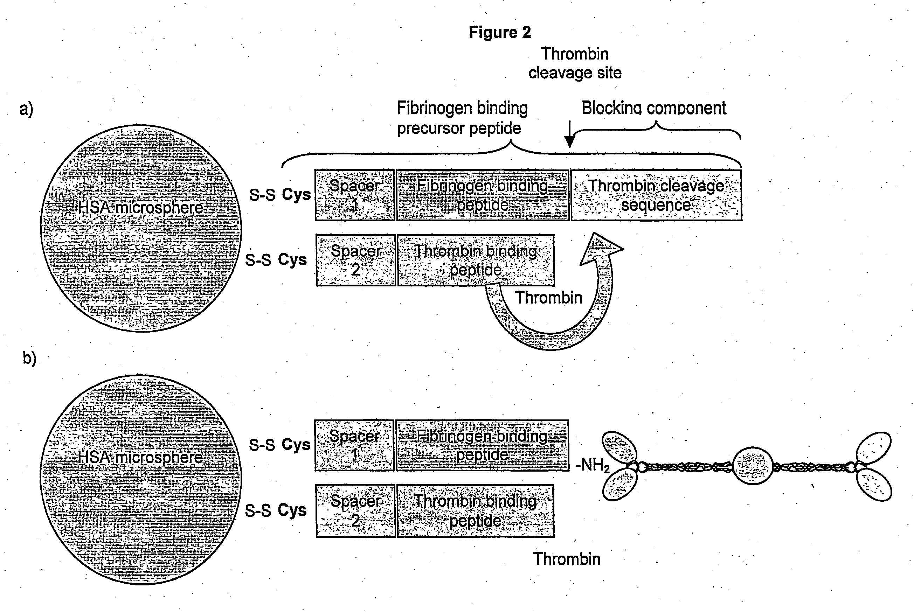

A second preferred embodiment of an agent of the invention is shown schematically in Figure 2(a). The agent comprises two separate peptides crosslinked to an HSA microsphere: a fibrinogen binding precursor peptide, and a thrombin binding peptide. The fibrinogen binding precursor peptide comprises (from its carboxy-terminal end to its amino terminal end): a carboxy-terminal cysteine residue which is cross-linked to the HSA microsphere; a first spacer sequence (Spacer 1); a fibrinogen binding peptide; and a thrombin cleavage sequence (the box in the figure labelled "Thrombin cleavage sequence" represents the sequence which is amino terminal to a site at which thrombin is able to cleave the fibrinogen binding precursor peptide, i.e. the thrombin cleavage site). The portion of the fibrinogen binding precursor peptide which is on the amino-terminal side of the fibrinogen binding peptide (i.e. the Thrombin cleavage sequence) forms a blocking component. The blocking component blocks or inhibits binding of fibrinogen to the fibrinogen binding peptide.

The thrombin binding peptide is linked at its carboxy-terminal end to a second spacer sequence (Spacer 2). The carboxy-terminal end of the second spacer sequence is linked to a cysteine residue which is cross-linked to the HSA microsphere.

Thrombin generated at a wound site binds to the thrombin binding peptide and cleaves the fibrinogen binding precursor peptide at the thrombin cleavage site. Cleavage of the fibrinogen binding precursor peptide releases the blocking component from the fibrinogen binding peptide thereby exposing the fibrinogen binding peptide and allowing binding of fibrinogen to the fibrinogen binding peptide (Figure 2(b)).

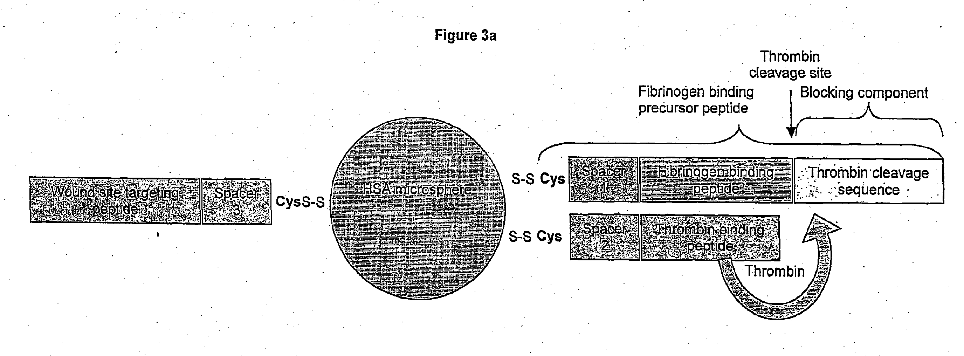

A third preferred embodiment of an agent of the invention is shown schematically in Figure 3a. The agent comprises three separate peptides crosslinked to an HSA microsphere: a fibrinogen binding precursor peptide, a thrombin binding peptide, and a wound site targeting peptide. The fibrinogen binding precursor peptide and the thrombin binding peptide are as described above for the second preferred embodiment. The wound site targeting peptide is linked at its carboxy-terminal end to a third spacer sequence (Spacer 3). The carboxy-terminal end of the third spacer sequence is linked to a cysteine residue which is cross-linked to the HSA microsphere. The wound site targeting peptide is able to bind selectively to a wound site (for example to cell surface protein tissue factor).

The wound site targeting peptide promotes localisation of the agent to a wound site. Thrombin generated at the wound site binds to the thrombin binding peptide and cleaves the fibrinogen binding precursor peptide at the thrombin cleavage site. Cleavage of the fibrinogen binding precursor peptide releases the blocking component from the fibrinogen binding peptide thereby exposing the fibrinogen binding peptide and allowing binding of fibrinogen to the fibrinogen binding peptide (Figure 3a).

The third spacer sequence spaces the wound site targeting peptide from the HSA microsphere so that the microsphere does not interfere with binding of the wound site targeting peptide to the wound site.

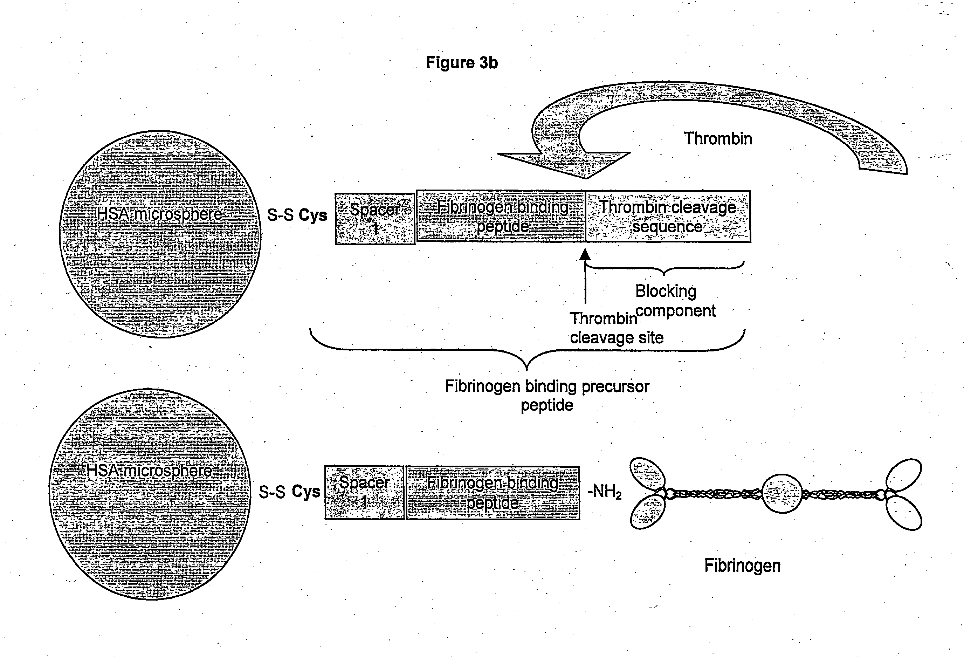

A fourth preferred embodiment of an agent of the invention is shown schematically in Figure 3b. The agent comprises a fibrinogen binding precursor peptide bound to a Human Serum Albumin (HSA) microsphere. The fibrinogen blinding precursor peptide comprises (from its carboxy-terminal end to its amino terminal end): a carboxy-terminal cysteine residue which is cross-linked to the HSA microsphere; a first spacer sequence (Spacer 1); a fibrinogen binding peptide; and a thrombin cleavage sequence (the box in the figure labelled "Thrombin cleavage sequence" represents the sequence which is amino terminal to the site at which thrombin is able to cleave the fibrinogen binding precursor peptide, i.e. the thrombin cleavage site).

The portion of the fibrinogen binding precursor peptide which is on the amino-terminal side of the fibrinogen binding peptide (i.e. the Thrombin cleavage sequence) forms a blocking component. The blocking component blocks or inhibits binding of fibrinogen to the fibrinogen binding peptide. Thrombin generated at a wound site is able to cleave the fibrinogen binding precursor peptide at the amino terminal end of the fibrinogen binding peptide (as shown in Figure 3b). Cleavage of the fibrinogen binding precursor peptide releases the blocking component from the fibrinogen binding peptide, thereby exposing the fibrinogen binding peptide and allowing binding of fibrinogen to the fibrinogen binding peptide (as shown in the lower part of Figure 3b).

Examples of suitable peptide sequences of the four preferred embodiments of agents of the invention described above and shown in Figures 1-3 are:
Spacer 1: NH₂-GGGGGG-COOH (SEQ ID NO: 29);
Fibrinogen binding peptide: NH₂-GPRP-COOH (SEQ ID NO: 17);
Thrombin cleavage sequence: NH₂-LVPR-COOH (SEQ ID NO: 31);
Spacer 2: a peptide sequence that allows thrombin to be optimally orientated to cleave the thrombin cleavage site when it is bound to the thrombin binding peptide;
Thrombin binding peptide: a peptide sequence derived from the thrombin exocite 1 binding site on a molecule capable of binding thrombin, such as PAR-1 (i.e. WEDEEKNES (SEQ ID NO: 24));
Wound site targeting peptide: Factor VII or a fragment or derivative thereof (such as recombinant Factor VIIa);
Spacer 3: NH₂-GGGGGG-COOH (SEQ ID NO: 29).

In the preferred embodiments described above, the peptides comprise a carboxy terminal cysteine residue which is cross-linked to the HSA microsphere. It will be appreciated that any suitable covalent linkage to the microsphere may be used instead, for example a covalent linkage resulting from reaction of a maleimide group attached to the peptide with a sulphydryl group of the microsphere.

The preferred embodiments described above (and some of the embodiments described in the examples below) comprise microspheres. It will be appreciated that other microparticles or suitable insoluble carriers may be used instead of microspheres.

### Example 1

### Examples of agents of the invention:

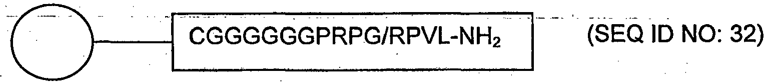 CGGGGGGPRPG/RPVL-NH2 (SEQ ID NO: 32)

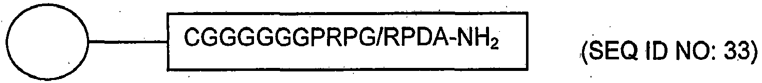 CGGGGGGPRPG/RPDA-NH2 (SEQ ID NO: 33)

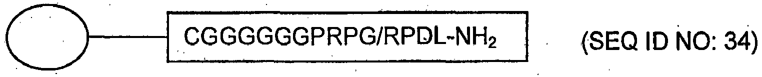 CGGGGGGPRPG/RPDL-NH2 (SEQ ID NO: 34)

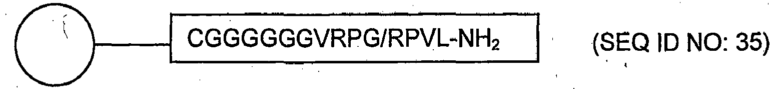 CGGGGGGVRPG/RPVL-NH2 (SEQ ID NO: 35)

where:
"/" represents a thrombin cleavage site;
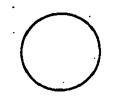 represents an albumin microsphere; and
- represents a covalent bond between the terminal cysteine of the peptide sequence and the albumin microsphere.
In this example, the surface of the albumin microspheres has been modified with Dithio-bis-(2-nitrobenzoic acid) (DTNB) to provide thiol-reactive groups on the carrier for reaction with the -SH groups of the carboxy-terminal cysteine residues of the peptides (see Example 2 below).

### Example 2

### Preparation of Fibrinogen Binding Precursor Peptide (Peptide A) and Fibrinogen Binding Peptide (Peptide B) bound to microspheres

H₂N-LVPRGPRPG⁶C (Peptide A) (SEQ ID NO: 36) and H₂N-GPRPG⁶C (Peptide B) (SEQ ID NO: 37) were each synthesised and cross-linked separately to microspheres composed of human albumin as follows:
25mg microspheres composed of human albumin were suspended in 0.02M Tris pH 7.5 at a final concentration of 10mg/ml;
75µl Dithio-bis-(2-nitrobenzoic acid) (DTNB) dissolved at a concentration of 4mg/ml in 0.2M sodium phosphate buffer pH 8.0, was added to the microspheres and mixed for 2hrs on a Dynal mixer;
After mixing to improve washing, an additional 2.5ml of 0.02M Tris pH 7.5 was added to tube, and mixed briefly by inversion;
The microspheres were separated from the supernatant by centrifugation at 1800g for 15minutes;
The microspheres were resuspended in 2.5ml of 0.02M Tris pH 7.5;
Peptide A or Peptide B was dissolved in 0.02M Tris pH 7.5 at a concentration of 10mg/ml. 100µl of peptide at 10mg/ml was added to the microspheres and mixed for 6hrs at room temperature on a Dynal mixer;
2.5ml of 0.02M Tris pH 7.5 was added and mixed briefly by inversion;
The microspheres were separated from the supernatant by centrifugation at 1800g for 15minutes;
The microspheres were washed with an additional 5.0ml 0.02M Tris pH 7.5, and separated by centrifugation as described;
The microspheres were resuspended in 2.5ml 0.02M Tris containing 5% Human serum albumin;
The microspheres were filled into 200µl aliquots and stored at -70°C.

### Example 3

### Measurement of binding of fibrinogen to Fibrinogen Binding precursor (Peptide A) and Fibrinogen Binding Peptide (Peptide B) linked microspheres (M/S), in the absence (i) and presence (ii) of thrombin

Peptide A linked microspheres, and Peptide B linked microspheres were prepared as described in Example 2, and were mixed for 30 minutes with buffer or thrombin as shown in the table below:

| Peptide | A | A | B | B |
|---|---|---|---|---|
| M/S (10mg/ml) | 50.µl | 50.µl | 50µl | 50µl |
| Thrombin (3.2 u/ml) | 0 | 5µl | 0 | 5µl |
| 0.02M Tris | 50µl | 45µl | 50µl | 45µl |
| Final thrombin concentration | 0 | 0.16 U/ml | 0 | 0.16U/ml |

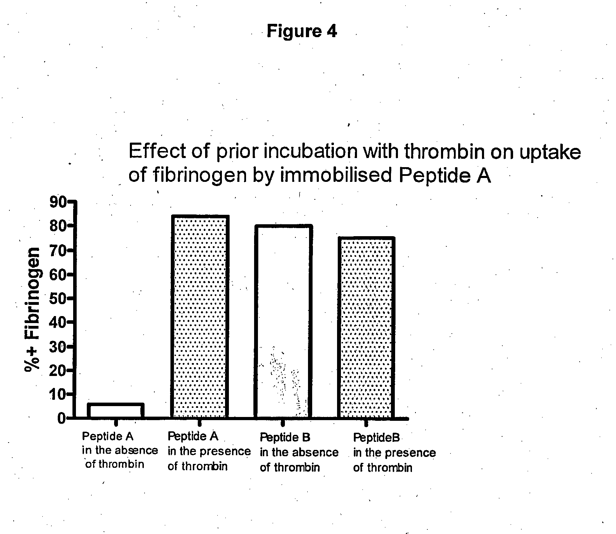

The microspheres were then recovered by centrifuging at 1800g for 10minutes. The microspheres were resuspended in 100µl Tris to give a final concentration of 5mg/ml. 10µl of Hirudin 100U/ml was added to inhibit residual thrombin.
180µl Fibrinogen at a concentration 0.1mg/ml was added to 20µl of Peptide A or Peptide B linked microspheres and mixed for 1hr.
Fibrinogen binding was measured as follows. 5µl microspheres were added to 50µl HEPES buffer pH 7.5. 1µl FITC labelled antibody to human fibrinogen was added and mixed for 20 minutes. The reaction was stopped with addition of 0.2% Formyl saline. Bound fluorescence was measured using a Beckman Coulter XL MCL Flow Cytometer.
The results are shown in Figure 4. When Peptide A microspheres were mixed with fibrinogen, those which had not previously been incubated with thrombin only bound a small amount of fibrinogen (6%). When Peptide A linked microspheres were first incubated with thrombin, and then mixed with fibrinogen, 86% bound fibrinogen.
Peptide B that was the fibrinogen binding peptide with no blocking sequence bound fibrinogen in the presence and absence of thrombin.

### Example 4

### Further examples of agents of the invention:

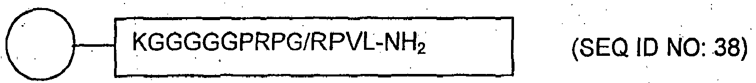 KGGGGGPRPG/RPVL-NH2 (SEQ ID NO: 38)

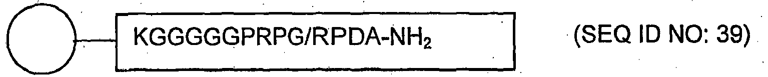 KGGGGGPRPG/RPDA-NH2 (SEQ ID NO: 39)

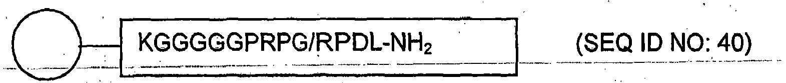 KGGGGGPRPG/RPDL-NH2 (SEQ ID NO: 40)

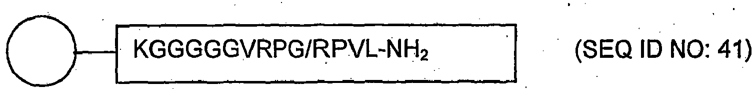 KGGGGGVRPG/RPVL-NH2 (SEQ ID NO: 41)

where:
"/" represents a thrombin cleavage site;
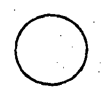 represents an albumin microparticle with surface sulphydryl groups; and represents a covalent thioether bond formed by reaction of a terminal maleimide of the peptide sequence and a sulphydryl group of the albumin microparticle.

### Example 5

### Linking of Fibrinogen Binding Precursor Peptide (Peptide C) to microparticles using maleimide linking chemistry

The following peptide was synthesised with a maleimide (MAL) moiety on the C-terminus H₂N-LVPRGPRPG⁵K-MAL (Peptide C) (SEQ ID NO: 42) and crosslinked to microparticles composed of human albumin as follows:
Prior to peptide conjugation the sulphydryl content of the microparticles was determined by a modified Ellmans assay as follows: Dithio-bis-(2-nitrobenzoic acid) (DTNB) was dissolved at a concentration of 4mg/ml in 0.1 M Na phosphate buffer pH 8 with 2 mM EDTA. The microparticle sample prepared at 20 mg/ml (0.2 ml) was mixed with 0.79 ml of 0.1 M Na phosphate buffer pH 8 with 2 mM EDTA and 10 µL of the DTNB stock was added to the reaction. The control blank was prepared with 0.2 ml of buffer without microparticles. The reactions were mixed on a turntable for 20 min then centrifuged (1800 g for 15 min) and the supernatant was collected by syringe and then filtered through a syringe filter of 0.22 µm. The filtrates were analysed using a spectrophotometer at 412 nm and the amount of sulphydryl groups was determined using a molar extinction coefficient of 13600. The thiol content was typically between 0.6 to 0.3 moles of thiol per mole of albumin protein.
For conjugation of maleimide modified peptide to microparticles; 62.5 mg of human serum albumin microparticles were dispersed in 2.4 ml of 0.1 M Tris/HCl pH 7 containing 2 mM EDTA by mixing on a turntable for 10 min followed by sonication for 10 min.
Peptide C was dissolved in 10 mM Na phosphate buffer pH 6.5 at 10 mg/ml. Peptide stock (100µl) was added to the microparticles and mixed for 1 h at room temperature on a Dynal mixer. The reaction was diluted in an equal volume of 0.02M Tris pH 7.5 and mixed briefly by inversion. The microparticles were separated from the supernatant by centrifugation at 1800g for 15min.
The microparticles were washed with an additional 5.0 ml 0.02M Tris/HCl pH 7.5, and separated by centrifugation as described;
The microparticles were resuspended in 2.5ml 0.02M Tris containing 2 % mannitol pH 7.5. The microparticles were filled into 200µl aliquots and stored at-70°C

### Example 6

### Measurement of fibrinogen binding to microparticles after incubation with or without thrombin

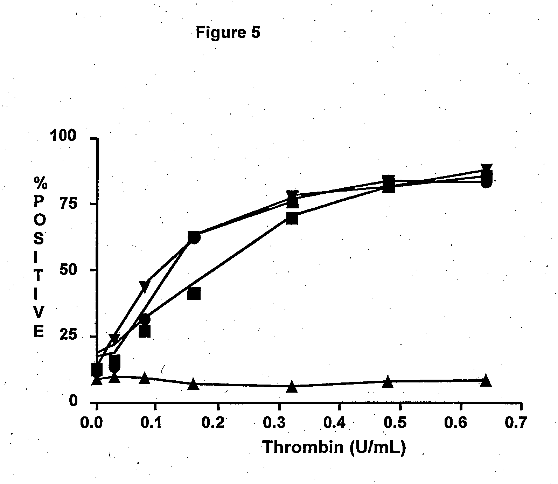

Microparticles were modified with peptide C as described in Example 5. Microparticles were mixed with fibrinogen solution after exposure to thrombin to confirm that fibrinogen-binding sequence is exposed by thrombin. Microparticles at 0.7, 5 or 10 mg/ml were incubated with 0-0.64 U/mL thrombin at 37°C after 2 min the thrombin was inhibited by the addition of hirudin (Sigma) at a final concentration of 10 U/mL. Microparticles were centrifuged at 9300 g for 5 min and resuspended in HBS to give 14 mg/mL. The microparticles were diluted 10-fold in 0.1mg/mL human fibrinogen (Scottish National Blood Transfusion Service: SNBTS) and incubated at room temperature for 1 h. The amount of fibrinogen bound was determined by flow cytometry with a polyclonal antibody rabbit-anti-human fibrinogen-FITC (Dako Cytomation). These results show in Figure 5 that the microparticles modified with peptide C without thrombin do not bind fibrinogen, but with increasing thrombin concentration the amount of fibrinogen bound to microparticles increased at all three concentrations of microparticles tested. Microparticles that were not modified with peptide C do not bind fibrinogen with or without thrombin exposure.

### Example 7

### Clot formation in fibrinogen in response to thrombin

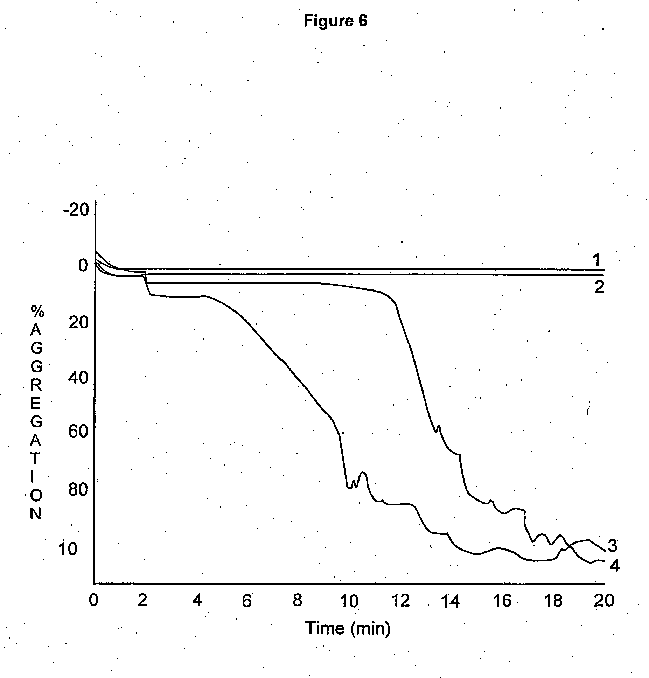

Clot formation was determined by a reduction in turbidity using the Platelet Aggregation Profiler® PAP-4 (BioData Corporation, PA) as follows;
Peptide C linked microparticles were prepared as described in Example 5 at a stock concentration of 2.5 mg/ml in Hepes buffered saline (HBS). The baseline optical density was set with fibrinogen (SNBTS) solution at 3 mg/ml in HBS.
Fibrinogen solutions (205 µl) were pre-warmed to 37°C for 1 min before the addition of microparticles (25 µl), with the stir bar rate set at 900 rpm. After two min 20 µL of human thrombin (Sigma) stock solution was added to give a final concentration of 0.032U/ml. A typical aggregation profile is shown in Figure 6. In the absence of thrombin neither blank nor microparticles modified with peptide C formed a clot. After the addition of thrombin at 2 min the turbidity of the solution decreased more rapidly for the solution containing the peptide C modified microparticles compared to control unmodified microparticles indicative of clot formation.

### Example 8

### Clot formation in plasma in response to thrombin after peptide C modified microparticles have been pre-incubated with thrombin

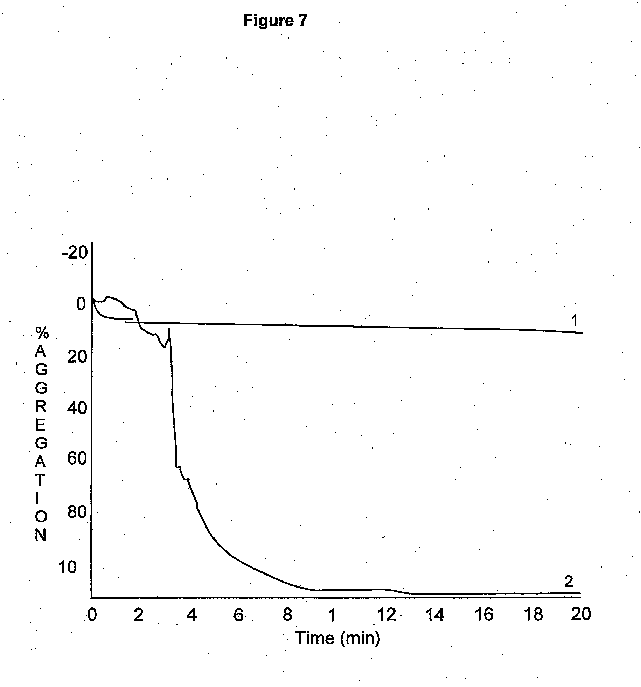

Clot formation as monitored in plasma with peptide C modified microparticles that had been pre-exposed to thrombin in a buffer. Microparticle clot formation in plasma was determined turbidimetrically using the Platelet Aggregation Profiler® PAP-4 (BioData Corporation, PA) as described above.
Peptide C modified microparticles were prepared as described in Example 5 at a stock concentration of 2.5 mg/ml in HBS. Peptide C modified microparticles were pre-incubated with 0.6 U/ml thrombin for 1h and then hirudin (10 U/ml) was added to inhibit thrombin activity. The reactions were centrifuged at 9300 g for 5 min then resuspended in HBS at 2.5 mg/ml. The baseline optical density was set with plasma, which had been prepared from citrated blood. Plasma samples (205 µl) were pre-warmed to 37°C for 1 min before the addition of microparticles (25 µl), with the stir bar rate set at 900 rpm. After two minutes 20 uL of thrombin stock solution was added to the reaction to give a final thrombin concentration of 0.25 U/ml. A typical aggregation profile is shown in Figure 7. This Figure shows that when the peptide C microparticles are exposed to thrombin both before and during incubation with plasma clot formation occurs. Without thrombin exposure the peptide C modified microparticles did not result in clot formation over the 20 min period.

### Example 9

### Clot formation in Plasma in response to thrombin

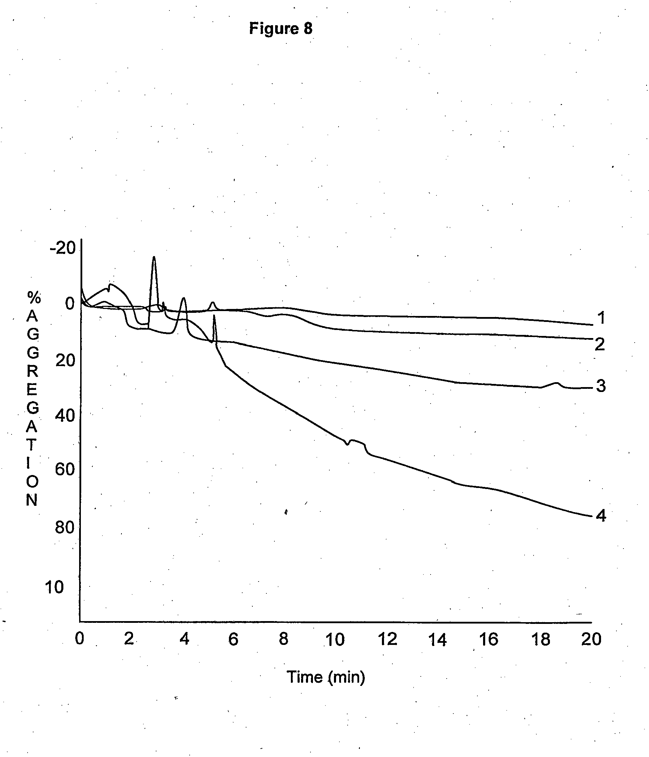

Microparticle clot formation in plasma was determined turbidimetrically using the Platelet Aggregation Profiler® PAP-4 (BioData Corporation, PA) as described above.
Peptide C modified microparticles were prepared as described in Example 5 at a stock concentration of 2.5 mg/ml in HBS. The baseline optical density was set with plasma, which had been prepared from citrated blood. Plasma samples (205 µl) were pre-warmed to 37°C for 1 min before the addition of microparticles (25 µl), with the stir bar rate set at 900 rpm. After two minutes 20 uL of human thrombin (Sigma) stock solution was added to the reaction to give a final thrombin concentration of 0.25 U/ml. A typical aggregation profile is shown in Figure 8. These results show that in plasma without the addition of thrombin both unmodified and peptide C modified microparticles did not form a clot. With the addition of thrombin at the 2 min time point the microparticles modified with Peptide C showed accelerated clot formation in plasma compared to unmodified control blank microparticles.

### Example 10

### Platelet aggregation in the presence of peptide C modified microparticles

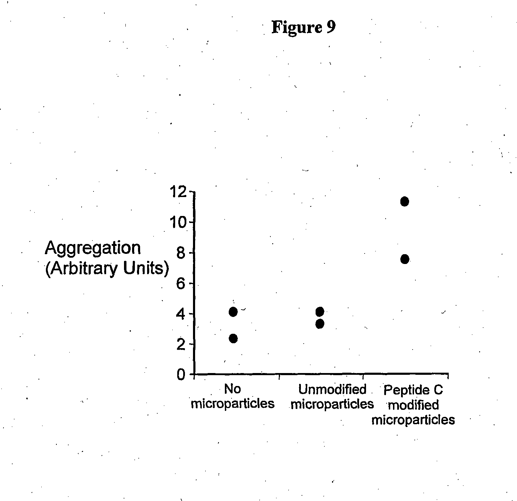

Platelet aggregation in the presence of peptide C modified microparticles was measured using a Multiplatelet impedance aggregometer (Multiplate® Dynabyte medical, Munich).
Peptide C modified microparticles were prepared as described in Example 5 at a stock concentration of 2.5 mg/ml in HBS. The microparticles (0.75 mg) were pre-incubated for 1 h with 1 U/ml human thrombin (Sigma) in HBS (total volume 0.3 ml). To inhibit thrombin activity hirudin (Sigma) was added at a final concentration of 5 U/ml. The reaction was centrifuged at 9300 g for 5 min and the supernatant was discarded. The microparticles were resuspended in HBS to a concentration of 2.5 mg/ml.
Thrombocytopenic blood was prepared from citrated blood as follows:
Platelets were removed by centrifuging whole blood at 163g for 20 min and discarding the platelet rich upper layer. The upper platelet rich layer was replaced by plasma that was isolated as the supernatant after centrifuging whole blood at 1800g for 30 min.
To monitor electrical impedance; to each electrode well 0.3 ml of blood was incubated at 37°C with microparticles (0.15 mg) and saline to give a total volume of 0.6 ml. Aggregation was recorded immediately after the addition of 30 µl of activator adenosine diphosphate solution (0.1 mM). Aggregation was recorded for 6 min; results in Figure 9 are expressed in arbitrary units of final aggregation. These results show greater electrical impedance (i.e. greater than final aggregation) in activated TCB in the presence of peptide C modified microparticles compared to TCB without microparticles or blank microparticles.

### Example 11

### Determining hydrolysis of candidate fibrinogen binding precursor peptides

Candidate peptides are synthesised with a radiolabel, chromogenic or fluorogenic molecule at the N-terminus of the peptide. The peptide is linked to an insoluble carrier using methods described in Example 2 or 5. The peptide-modified carrier is incubated with a pure protease in buffer. The amount of label remaining on the modified carrier allows the rate of hydrolysis of the peptide to be determined. For example, 5,6-Carboxyfluorescein labelled peptide 5,6-FAM-LVPRGPRPGGGGG-Cys (SEQ ID NO: 43). The labelled peptide is conjugated to albumin microparticles using the methods described in Example 2 using DNTB to facilitate peptide linking. The rate of peptide hydrolysis is determined by the amount of bound fluorescence remaining on the microparticle using a flow cytometer.
Microparticles conjugated with labelled peptide are mixed with various concentrations of human thrombin (Sigma) at pH 7.4 and incubated for various periods at 37°C. The reactions are then stopped by the addition of hirudin in a five-fold excess to thrombin units and the reactions are diluted appropriately to be analysed by flow cytometry. This procedure allows the identification of peptides that can be hydrolysed by thrombin when conjugated to albumin microparticles. It will be appreciated that equivalent procedures may be used to identify peptides that can be hydrolysed by other proteases when conjugated to microphseres.

### Example 12

### Determining the stability of fibrinogen binding precursor peptides

A candidate fibrinogen binding precursor peptide is synthesised with an N-terminal label and conjugated to an insoluble carrier as described in Example 11. The labelled peptide linked microparticle is incubated in a biological sample such as whole blood, plasma or tissue homogenate. Stability of the peptide reflects the amount of intact fluorescent peptide on the microparticle. To identify the protease involved a specific protease inhibitor is included in the incubate.
For example citrated blood is mixed with thrombin inhibitor hirudin (5 U/ml) and the blood is re-calcified with 20 mmol/mL of calcium. The microparticles modified with the labelled peptide are then added to the blood at a final concentration of 2.5 mg/ml. The reaction is mixed while being maintained at 37°C, samples of the reaction are taken at different time points, diluted appropriately and applied to a flow cytometer to determine the amount of intact bound peptide.

### Example 13

### Evaluation of dose related haemostatic activity in vivo

### 1. Dose related haemostatic activity in a thrombocytopenic rabbit model

Male New Zealand white rabbits 2.5-4.0kg are obtained from a reputable supplier. Groups of up to six rabbits are rendered thrombocytopenic using two doses of the cytotoxic drug Busulphan, 12 and 9 days respectively, prior to the study day. The dose of Busulphan is varied according to the severity of thrombocytopenia required, e.g. two doses of 20mg/kg will generally reduce the platelet count to between 10-20x10⁹/l, whereas two doses of 25mg/kg will reduce the platelet count to less than 10x10⁹/l. In addition to a reduction in platelet count, Busulphan dosing is associated with depletion of white cells, but only a minor reduction in haematocrit and no overt toxicity. No anaesthetic is required for this procedure.

Human platelet concentrates are used as a positive control for these studies. This requires only one platelet concentrate per group of animals. It has been shown previously that human platelets circulate for only approximately 5 minutes in the rabbit, due to uptake by the reticulo-endothelial system. Therefore in these experiments macrophage function is inhibited by dosing the rabbits with ethyl palmitate 24 hours before the study day. For consistency, treatment with ethyl palmitate can be used for all groups of animals.

On the study day the test agent is infused intravenously into an ear vein. Efficacy is assessed by measurement of bleeding time, which is performed using a standard incision in the ear

Variability in the bleeding time is controlled, as far as possible, by ensuring that the animals are quiet and warm and at an even temperature (Roskam, 1993, Comptes Rendus des Séances de la Societé de Biologie, 114, 166-169), and the number of blood samples minimised. Blajchman & Lee, 1997, Transfusion Medical Reviews, 11, 99-105. Bleeding times are measured immediately prior to administration of the test dose and at time points, up to 24 hours after dosing. Bleeding times in excess of 20 minutes are stopped by applying pressure to the wound. Animals are sacrificed at the completion of the study.

Dose related activity of an agent of the invention on a dose/kg basis is defined by reduction in bleeding time compared to the activity of human platelets. HSA micro particles of the same size but with no coupled peptide are used as the negative control. A comparison of the duration of effect over the 24 hour period of the study can be made.

In a thrombocytopenic rabbit, a bleeding time of about 20 minutes is typical. Agents of the invention should generally be able to reduce the bleeding time to less than 10 minutes in a minimum of three, and preferably all six, of the test rabbits in a group of six.

### 2. Assessment of thrombogenicity in a rabbit Wessler model

The potential thrombogenicity of an agent of the invention is assessed in the Wessler model, essentially as described by Wessler et al, 1959, Journal of Applied Physiology, 14, 943-946.

Male New Zealand White rabbits, body weight 2.5kg-4.0kg are obtained from an approved supplier and groups of up to six rabbits are anaesthetised. Segments of the right and left jugular veins are exposed and detached from the surrounding tissue. The test preparations are administered through an ear vein and following a period of circulation of 3 minutes the segments of the jugular veins are ligatured and left in situ for a further 10 minutes. The segments are carefully excised, and the lumen exposed. The vessel is examined for the presence of developed thrombi, which is scored visually.

Agents of the invention should not produce thrombi at doses which are 5-fold, preferably 10-fold, higher than the dose associated with optimal reduction in bleeding time.

A suitable dose of an agent of the invention may be 1x10⁸ to 2x10¹⁰ product particles per kg of patient body weight. For example, the dose (when expressed as number of product particles per kg body weight) may be about 2x10⁸, 3x 10⁸, 4x 10⁸, 5x 10⁸, 6x 10⁸, 7x 10⁸, 8x10⁸, 9x10⁸, 1x10⁹, 2x10⁹, 3x10⁹, 4x10⁹, 5x 10⁹, 6x10⁹, 7x10⁹, 8x10⁹, 9x10⁹, 1x10¹⁰ or 2x10¹⁰.

A suitable dose may also be expressed as milligrams of total protein per kg patient body weight. On this basis a suitable dose may be 5-200 mg/kg. For example, the dose may be about 5, 10, 15, 20, 30, 40, 50, 60, 70, 80, 90, 150 or 200 mg/kg.

An ideal dose has a safety margin of at least two-fold, preferably about 10-fold. In other words, the ideal dose is effective but remains safe even when increased by two-fold or about 10-fold. A safe dose does not form a clot using the Wessler test as described above.

## Claims

1. An agent which comprises a fibrinogen binding precursor bound to an insoluble carrier, wherein the fibrinogen binding precursor is a peptide that is covalently bound to the insoluble carrier, wherein the peptide consists of:
a carboxy-terminal amino acid residue, or a modified carboxy-terminal amino acid residue, which is covalently bound to the insoluble carrier;
a fibrinogen binding peptide of 4 to 30 amino acid residues in length;
a blocking peptide of 1 to 30 amino acid residues in length joined to the amino terminal end of the fibrinogen binding peptide that blocks or inhibits binding of fibrinogen to the fibrinogen binding peptide;
a cleavage site, that is recognised specifically by thrombin, and which is located between the fibrinogen binding peptide and the blocking peptide; and optionally
a spacer sequence of 1 to 20 amino acid residues in length between the carboxy-terminal residue, or the modified carboxy-terminal residue, and the fibrinogen binding peptide;
wherein cleavage of the fibrinogen binding precursor peptide by thrombin exposes the fibrinogen binding peptide bound to the carrier, with the amino acid sequence NH₂-G(P,H)RX- (SEQ ID NO: 16) at its amino terminal end, wherein X is any amino acid and wherein the fibrinogen binding peptide has increased ability, compared to the fibrinogen binding precursor peptide, to bind fibrinogen.

2. An agent according to claim 1, wherein the fibrinogen binding peptide exposed following cleavage of the fibrinogen binding precursor peptide comprises the amino acid sequence NH₂-GPRP- (SEQ ID NO: 17) at its amino terminal end.

3. An agent according to claim 1 or 2, wherein the fibrinogen binding precursor peptide comprises a peptide which includes the amino acid sequence NH₂-ZYXR/GPRP- (SEQ ID NO: 18) at its amino terminal end, where "*I*" represents the thrombin cleavage site, and X is any amino acid but is preferably proline, Y is any amino acid but is preferably aspartic acid, and Z is at least one amino acid that is preferably leucine or proline.

4. An agent according to claim 3, wherein the peptide comprises one of the following amino acid sequences at its amino terminal end: NH₂-LVPR/GPRP-(SEQ ID NO: 19), NH₂-ADPR/GPRP- (SEQ ID NO: 20) or NH₂-LDPR/GPRP-(SEQ ID NO: 21) where "/" represents the thrombin cleavage site.

5. An agent according to claim 3 or 4, which further comprises a thrombin binding site, wherein the fibrinogen binding precursor peptide and the thrombin binding site are bound separately to the insoluble carrier.

6. An agent according to claim 5, wherein the thrombin binding site is a peptide sequence to which thrombin can bind, preferably a sequence to which a thrombin exocite I or II domain can bind.

7. An agent according to claim 6, wherein the peptide sequence corresponds to peptide sequence of the PAR-1 receptor (such as WEDEEKNES (SEQ ID NO: 24)), or Factor VIII (such as EEEDWD (SEQ ID NO: 26) or EDSYED (SEQ ID NO: 27)) to which a thrombin exocite I or II domain can bind, or to the thrombin binding peptide hirudin.

8. An agent according to any preceding claim, wherein the carboxy-terminal residue is a cysteine residue, or wherein the modified carboxy-terminal residue is a maleimide-modified lysine residue.

9. An agent according to any preceding claim, wherein the spacer sequence is GGGGGG (SEQ ID NO: 29) or GGGGG (SEQ ID NO: 30).

10. An agent according to any preceding claim which further comprises a wound site targeting component immobilised to the insoluble carrier.

11. An agent according to claim 10, wherein the wound site targeting component is capable of binding to cell surface protein tissue factor.

12. An agent according to claim 11, wherein the wound site targeting component comprises Factor VII or a fragment or derivative thereof capable of binding cell surface protein tissue factor, or Factor VIIa or a fragment or derivative thereof capable of binding cell surface protein tissue factor.

13. A pharmaceutical composition comprising an agent as defined in any of claims 1 to 12, and a pharmaceutically acceptable carrier, excipient, or diluent.

14. An agent as defined in any of claims 1 to 12 for use as a medicament.

15. Use of an agent as defined in any of claims 1 to 12 in the manufacture of a medicament for preventing, treating, or ameliorating thrombocytopenia or thrombasthenia.

16. An agent as defined in any of claims 1 to 12 for preventing, treating, or ameliorating thrombocytopenia or thrombasthenia.

17. A fibrinogen binding precursor peptide for use as part of an agent according to claim 1, comprising the amino acid sequence NH₂-ZYXR/GPRP-(SEQ ID NO: 18) at its amino terminal end, where "/" represents a thrombin cleavage site, and X is proline, Y is aspartic acid or alanine, and Z is leucine or proline.

18. A peptide which comprises an amino acid sequence of any of SEQ ID NOs: 32-35, or 38-41.

19. A method of identifying a fibrinogen binding precursor peptide for use as part of an agent according to claim 1, which comprises:
i) incubating a wound site specific serine protease with a labelled candidate fibrinogen binding precursor peptide bound to an insoluble carrier under conditions that permit cleavage of known fibrinogen binding precursor peptides to fibrinogen binding peptides by the wound site specific serine protease;
ii) determining the amount of label on the insoluble carrier before and after incubation with the wound site specific serine protease; and
iii) identifying the candidate fibrinogen binding precursor peptide as a fibrinogen binding precursor peptide if the amount of label on the insoluble carrier after incubation with the wound site specific serine protease is less than the amount of label on the insoluble carrier before incubation with the wound site specific serine protease.

20. A method according to claim 19, which further comprises determining binding of fibrinogen to the insoluble carrier before and after incubation with the wound site specific serine protease, and in step (iii) identifying the candidate fibrinogen binding precursor peptide as a fibrinogen binding precursor peptide if the amount of label on the insoluble carrier after incubation with the wound site specific serine protease is less than the amount of label on the insoluble carrier before incubation with the wound site specific serine protease, and the binding of fibrinogen to the insoluble carrier is increased after incubation with the wound site specific serine protease.

21. A method of identifying a fibrinogen binding precursor peptide for use as part of an agent according to claim 1, which comprises:
i) contacting a wound site specific serine protease with a candidate fibrinogen binding precursor peptide bound to an insoluble carrier under conditions that permit cleavage of fibrinogen binding precursor peptides to fibrinogen binding peptides by the wound site specific serine protease;
ii) determining whether the candidate fibrinogen binding precursor peptide bound to the insoluble carrier promotes clot formation or platelet aggregation after it has been contacted with the wound site specific serine protease; and
iii) identifying the candidate fibrinogen binding precursor peptide as a fibrinogen binding precursor peptide if clot formation or platelet aggregation is promoted after the candidate fibrinogen binding precursor has been contacted with the wound site specific serine protease, but minimal clot formation or platelet aggregation is promoted prior to contact with the wound site specific serine protease.

## Patentansprüche

1. Mittel, das einen Fibrinogen-bindenden Vorläufer umfasst, der an einen unlöslichen Träger gebunden ist, wobei der Fibrinogen-bindende Vorläufer ein Peptid ist, das kovalent an den unlöslichen Träger gebunden ist, wobei das Peptid besteht aus:
einem Carboxy-terminalen Aminosäurerest oder einem modifizierten Carboxy-terminalen Aminosäurerest, der kovalent an den löslichen Träger gebunden ist;
einem Fibrinogen-bindenden Peptid mit einer Länge von 4 bis 30 Aminosäureresten;
einem Blockierungspeptid mit einer Länge von 1 bis 30 Aminosäureresten, das an das aminoterminale Ende des Fibrinogen-bindenden Peptids gefügt ist und die Bindung von Fibrinogen an das Fibrinogen-bindende Peptid blockiert oder inhibiert;
einer Spaltungsstelle, die spezifisch von Thrombin erkannt wird und die sich zwischen dem Fibrinogen-bindenden Peptid und dem Blockierungspeptid befindet; und optional
einer Abstandshaltersequenz mit einer Länge von 1 bis 20 Aminosäureresten zwischen dem Carboxy-terminalen Rest oder dem modifizierten Carboxy-terminalen Rest und dem Fibrinogen-bindenden Peptid;
wobei die Spaltung des Fibrinogen-bindenden Vorläuferpeptids durch Thrombin das an den Träger gebundene Fibrinogen-bindende Peptid exponiert, mit der Aminosäuresequenz NH₂-G(P,H)RX- (SEQ ID Nr. 16) an seinem aminoterminalen Ende, wobei X eine beliebige Aminosäure ist und wobei das Fibrinogen-bindende Peptid eine höhere Fibrinogenbindungsfähigkeit hat als das Fibrinogen-bindende Vorläuferpeptid.

2. Mittel nach Anspruch 1, wobei das nach der Spaltung des Fibrinogen-bindenden Vorläuferpeptids exponierte Fibrinogen-bindende Peptid die Aminosäuresequenz NH₂-GPRP-(SEQ ID Nr. 17) an seinem aminoterminalen Ende umfasst.

3. Mittel nach Anspruch 1 oder 2, wobei das Fibrinogen-bindende Vorläuferpeptid ein Peptid umfasst, das die Aminosäuresequenz NH₂-ZYXR/GPRP- (SEQ ID Nr. 18) an seinem aminoterminalen Ende hat, wobei "/" die Thrombinspaltungsstelle repräsentiert und X eine beliebige Aminosäure, aber vorzugsweise Prolin ist, Y eine beliebige Aminosäure, aber vorzugsweise Asparaginsäure ist, und Z wenigstens eine Aminosäure ist, die vorzugsweise Leucin oder Prolin ist.

4. Mittel nach Anspruch 3, wobei das Peptid eine der folgenden Aminosäuresequenzen an seinem aminoterminalen Ende umfasst: NH₂-LVPR/GPRP- (SEQ ID Nr. 19), NH₂-ADPR/GPRP-(SEQ ID Nr. 20) oder NH₂-LDPR/GPRP- (SEQ ID Nr. 21), wobei "/" die Thrombinspaltungsstelle repräsentiert.

5. Mittel nach Anspruch 3 oder 4, das ferner eine Thrombinbindungsstelle umfasst, wobei das Fibrinogen-bindende Vorläuferpeptid und die Thrombinbindungsstelle separat an den unlöslichen Träger gebunden sind.

6. Mittel nach Anspruch 5, wobei die Thrombinbindungsstelle eine Peptidsequenz ist, an die sich Thrombin binden kann, vorzugsweise eine Sequenz, an die sich eine Thrombin Exocite I oder II Domäne binden kann.

7. Mittel nach Anspruch 6, wobei die Peptidsequenz einer Peptidsequenz des PAR-1 Rezeptors (wie z.B. WEDEEKNES (SEQ ID Nr. 24)) oder Faktor VIII (wie z.B. EEEDWD (SEQ ID Nr. 26) oder EDSYED (SEQ ID Nr. 27)) entspricht, an die sich eine Thrombin Exocite I oder II Domäne binden kann, oder an das Thrombin-bindende Peptid Hirudin.

8. Mittel nach einem vorherigen Anspruch, wobei der Carboxy-terminale Rest ein Cysteinrest ist oder wobei der modifizierte Carboxy-terminale Rest ein Maleimidmodifizierter Lysinrest ist.

9. Mittel nach einem vorherigen Anspruch, wobei die Abstandshaltersequenz GGGGGG (SEQ ID Nr. 29) oder GGGGG (SEQ ID Nr. 30) ist.

10. Mittel nach einem vorherigen Anspruch, das ferner eine Wundstellentargetierungskomponente umfasst, die auf dem unlöslichen Träger immobilisiert ist.

11. Mittel nach Anspruch 10, wobei sich die Wundstellentargetierungskomponente an Zelloberflächen-Proteingewebefaktor binden kann.

12. Mittel nach Anspruch 11, wobei die Wundstellentargetierungskomponente Faktor VII oder ein Fragment oder Derivat davon umfasst, das Zelloberflächen-Proteingewebefaktor oder Faktor VIIa oder ein Fragment oder Derivat davon binden kann, das Zelloberflächen-Proteingewebefaktor binden kann.

13. Pharmazeutische Zusammensetzung, die ein Mittel nach einem der Ansprüche 1 bis 12 und ein(en) pharmazeutisch akteptablen/s Träger, Hilfsstoff oder Verdünnungsmittel umfasst.

14. Mittel nach einem der Ansprüche 1 bis 12 zur Verwendung als Medikament.

15. Verwendung eines Mittels nach einem der Ansprüche 1 bis 12 bei der Herstellung eines Medikaments zur Verhütung, Behandlung oder Linderung von Thrombozytopenie oder Thrombasthenie.

16. Mittel nach einem der Ansprüche 1 bis 12 zum Verhüten, Behandeln oder Lindern von Thrombozytopenie oder Thrombasthenie.

17. Fibrinogen-bindendes Vorläuferpeptid zur Verwendung als Teil eines Mittels nach Anspruch 1, das die Aminosäuresequenz NH₂-ZYXR/GPRP- (SEQ ID Nr. 18) an ihrem aminoterminalen Ende umfasst, wobei "/" eine Thrombinspaltungsstelle repräsentiert und X Prolin ist, Y Asparaginsäure oder Alanin ist und Z Leucin oder Prolin ist.

18. Peptid, das eine Aminosäuresequenz nach einem der SEQ ID Nrn. 32-35 oder 38-41 umfasst.

19. Verfahren zum Identifizieren von Fibrinogen-bindendem Vorläuferpeptid zur Verwendung als Teil eines Mittels nach Anspruch 1, das Folgendes beinhaltet:
i) Inkubieren einer wundstellenspezifischen Serinprotease mit einem markierten Fibrinogen-bindenden Kandidaten-Vorläuferpeptid, das an einen unlöslichen Träger unter Bedingungen gebunden ist, die eine Spaltung von bekannten Fibrinogen-bindenden Vorläuferpeptiden an Fibrinogen-bindende Peptide mit der wundstellenspezifischen Serinprotease zulassen;
ii) Ermitteln der Markermenge auf dem unlöslichen Träger vor und nach der Inkubation mit der wundstellenspezifischen Serinprotease; und
iii) Identifizieren des Fibrinogen-bindenden Kandidaten-Vorläuferpeptids als Fibrinogen-bindendes Vorläuferpeptid, wenn die Markermenge auf dem unlöslichen Träger nach der Inkubation mit der wundstellenspezifischen Serinprotease kleiner ist als die Markermenge auf dem unlöslichen Träger vor der Inkubation mit der wundstellenspezifischen Serinprotease.

20. Verfahren nach Anspruch 19, das ferner das Ermitteln der Bindung von Fibrinogen an den unlöslichen Träger vor und nach der Inkubation mit der wundstellenspezifischen Serinprotease beinhaltet, und in Schritt iii) das Identifizieren des Fibrinogen-bindenden Kandidaten-Vorläuferpeptids als Fibrinogen-bindendes Vorläuferpeptid, wenn die Markermenge an dem unlöslichen Träger nach der Inkubation mit der wundstellenspezifischen Serinprotease kleiner ist als die Markermenge an dem unlöslichen Träger vor der Inkubation mit der wundstellenspezifischen Serinprotease, und die Bindung von Fibrinogen an den unlöslichen Träger nach der Inkubation mit der wundstellenspezifischen Serinprotease erhöht wird.

21. Verfahren zum Identifizieren von Fibrinogen-bindendem Vorläuferpeptid zur Verwendung als Teil eines Mittels nach Anspruch 1, das Folgendes beinhaltet:
i) Kontaktieren einer wundstellenspezifischen Serinprotease mit einem Fibrinogen-bindenden Kandidaten-Vorläuferpeptid, das an einen unlöslichen Träger unter Bedingungen gebunden ist, die eine Spaltung von Fibrinogen-bindenden Vorläuferpeptiden an Fibrinogen-bindende Peptide durch die wundstellenspezifische Serinprotease zulassen;
ii) Ermitteln, ob das Fibrinogen-bindende Kandidaten-Vorläuferpeptid, das an den unlöslichen Träger gebunden ist, Blutgerinnselbildung oder Blutplättchenaggregation fördert, nachdem es mit der wundstellenspezifischen Serinprotease kontaktiert wurde; und
iii) Identifizieren des Fibrinogen-bindenden Kandidaten-Vorläuferpeptids als Fibrinogen-bindendes Vorläuferpeptid, wenn Gerinnselbildung oder Blutplättchenaggregation nach dem Kontaktieren des Fibrinogen-bindenden Kandidatenvorläufers mit der wundstellenspezifischen Serinprotease gefördert wird, aber minimale Gerinnselbildung oder Blutplättchenaggregation vor dem Kontakt mit der wundstellenspezifischen Serinprotease gefördert wird.

## Revendications

1. Agent comprenant un précurseur de liaison au fibrinogène lié à un support insoluble, dans lequel le précurseur de liaison au fibrinogène est un peptide lié de façon covalente au support insoluble, le peptide comprenant :
un résidu d'acide aminé à terminaison carboxy, ou un résidu d'acide aminé à terminaison carboxy modifié, qui est lié de façon covalente au support insoluble ;
un peptide de liaison au fibrinogène d'une longueur de 4 à 30 résidus d'acides aminés ;
un peptide de blocage d'une longueur de 1 à 30 résidus d'acides aminés rattaché à la terminaison amino du peptide de liaison au fibrinogène qui bloque ou inhibe la liaison du fibrinogène au peptide de liaison au fibrinogène ;
un site de clivage, qui est spécifiquement reconnu par la thrombine, et qui est situé entre le peptide de liaison au fibrinogène et le peptide de blocage ; et facultativement
une séquence d'espacement d'une longueur de 1 à 20 résidus d'acides aminés entre le résidu à terminaison carboxy, ou le résidu à terminaison carboxy modifié, et le peptide de liaison au fibrinogène ;
dans lequel le clivage du peptide précurseur de liaison au fibrinogène par la thrombine expose le peptide de liaison au fibrinogène lié au support, avec la séquence d'acides aminés NH₂-G(P,H)RX- (SEQ ID n° : 16) au niveau de son extrémité amino-terminale, où X est un quelconque acide aminé et dans lequel le peptide de liaison au fibrinogène a une meilleure capacité de liaison au fibrinogène que le peptide précurseur de liaison au fibrinogène.

2. Agent selon la revendication 1, dans lequel le peptide de liaison au fibrinogène exposé suite au clivage du peptide précurseur de liaison au fibrinogène comprend la séquence d'acides aminés NH₂-GPRP- (SEQ ID n° : 17) au niveau de sa terminaison amino.

3. Agent selon la revendication 1 ou 2, dans lequel le peptide précurseur de liaison au fibrinogène comprend un peptide qui inclut la séquence d'acides aminés NH₂-ZYXR/GPRP-(SEQ ID n° : 18) au niveau de sa terminaison amino, où « / » représente le site de clivage par la thrombine, et X est un quelconque acide aminé mais est de préférence la proline, Y est un quelconque acide aminé mais est de préférence l'acide aspartique et Z est au moins un acide aminé qui est de préférence la leucine ou la proline.

4. Agent selon la revendication 3, dans lequel le peptide comprend l'une des séquences d'acides aminés suivantes au niveau de sa terminaison amino : NH₂-LVPR/GPRP- (SEQ ID n° : 19), NH₂-ADPR/GPRP- (SEQ ID n° : 20) ou NH₂-LDPR/GPRP-(SEQ ID n° : 21), où « / » représente le site de clivage par la thrombine.

5. Agent selon la revendication 3 ou 4, qui comprend en outre un site de liaison à la thrombine, dans lequel le peptide précurseur de liaison au fibrinogène et le site de liaison à la thrombine sont liés séparément au support insoluble.

6. Agent selon la revendication 5, dans lequel le site de liaison à la thrombine est une séquence peptidique à laquelle la thrombine peut se lier, de préférence une séquence à laquelle un domaine de l'exosite I ou II de la thrombine peut se lier.

7. Agent selon la revendication 6, dans lequel la séquence peptidique correspond à une séquence peptidique du récepteur PAR-1 (par exemple WEDEEKNES (SEQ ID n° : 24)) ou du Facteur VIII (par exemple EEEDWD SEQ ID n° : 26) ou EDSYED (SEQ ID n° : 27)) à laquelle un domaine de l'exosite I ou II de la thrombine peut se lier, ou à l'hirudine du peptide de liaison à la thrombine.

8. Agent selon l'une quelconque des revendications précédentes, dans lequel le résidu à terminaison carboxy est un résidu de cystéine, ou dans lequel le résidu à terminaison carboxy modifié est un résidu de lysine modifié par maléimide.

9. Agent selon l'une quelconque des revendications précédentes, dans lequel la séquence d'espacement est GGGGGG (SEQ ID n° : 29) ou GGGGG (SEQ ID n° : 30).

10. Agent selon l'une quelconque des revendications précédentes, qui comprend en outre un composant de ciblage du site de lésion immobilisé sur le support insoluble.

11. Agent selon la revendication 10, dans lequel le composant de ciblage du site de lésion est capable de se lier au facteur tissulaire de la protéine de surface cellulaire.

12. Agent selon la revendication 11, dans lequel le composant de ciblage du site de lésion comprend le Facteur VII ou son fragment ou dérivé, capable de se lier au facteur tissulaire de la protéine de surface cellulaire, ou le Facteur VIIa ou son fragment ou dérivé, capable de se lier au facteur tissulaire de la protéine de surface cellulaire.

13. Composition pharmaceutique comprenant un agent tel que défini dans l'une quelconque des revendications 1 à 12, et un support, excipient ou diluant pharmaceutiquement acceptable.

14. Agent tel que défini dans l'une quelconque des revendications 1 à 12, pour une utilisation comme médicament.

15. Utilisation d'un agent tel que défini dans l'une quelconque des revendications 1 à 12, dans la fabrication d'un médicament pour prévenir, traiter ou améliorer la thrombocytopénie ou la thrombasthénie.

16. Agent tel que défini dans l'une quelconque des revendications 1 à 12, pour prévenir, traiter ou améliorer la thrombocytopénie ou la thrombasthénie.

17. Peptide précurseur de liaison au fibrinogène pour une utilisation dans le cadre d'un agent selon la revendication 1, comprenant la séquence d'acides aminés NH₂-ZYXR/GPRP-(SEQ ID n° : 18) au niveau de sa terminaison amino, où « / » représente un site de clivage par la thrombine, et X est la proline, Y est l'acide aspartique ou l'alanine et Z est la leucine ou la proline.

18. Peptide qui comprend une séquence d'acides aminés de l'une quelconque de SEQ ID n° : 32 à 35 ou 38 à 41.

19. Procédé d'identification d'un peptide précurseur de liaison au fibrinogène pour une utilisation dans le cadre d'un agent selon la revendication 1, qui comprend les étapes consistant à :
i) incuber une protéase à sérine spécifique du site de lésion avec un peptide précurseur de liaison au fibrinogène candidat marqué lié à un support insoluble dans des conditions permettant le clivage des peptides précurseurs de liaison au fibrinogène connus et des peptides de liaison au fibrinogène par la protéase à sérine spécifique du site de lésion ;
ii) déterminer la quantité de marqueur sur le support insoluble avant et après incubation avec la protéase à sérine spécifique du site de lésion ; et
iii) identifier le peptide précurseur de liaison au fibrinogène candidat sous forme de peptide précurseur de liaison au fibrinogène si la quantité de marqueur sur le support insoluble après incubation avec la protéase à sérine spécifique du site de lésion est inférieure à la quantité de marqueur sur le support insoluble avant incubation avec la protéase à sérine spécifique du site de lésion.

20. Procédé selon la revendication 19, qui comprend en outre la détermination de la liaison du fibrinogène au support insoluble avant et après incubation avec la protéase à sérine spécifique du site de lésion, et dans l'étape (iii) l'identification du peptide précurseur de liaison au fibrinogène candidat sous forme de peptide précurseur de liaison au fibrinogène si la quantité de marqueur sur le support insoluble après incubation avec la protéase à sérine spécifique du site de lésion est inférieure à la quantité de marqueur sur le support insoluble avant incubation avec la protéase à sérine spécifique du site de lésion, et la liaison du fibrinogène au support insoluble est améliorée après incubation avec la protéase à sérine spécifique du site de lésion.

21. Procédé d'identification d'un peptide précurseur de liaison au fibrinogène pour une utilisation dans le cadre d'un agent selon la revendication 1, qui comprend les étapes consistant à :
i) mettre en contact une protéase à sérine spécifique du site de lésion avec un peptide précurseur de liaison au fibrinogène candidat lié à un support insoluble dans des conditions permettant le clivage des peptides précurseurs de liaison au fibrinogène et des peptides de liaison au fibrinogène par la protéase à sérine spécifique du site de lésion ;
ii) déterminer si le peptide précurseur de liaison au fibrinogène candidat lié au support insoluble favorise la formation de caillots ou l'agrégation plaquettaire après avoir été mis en contact avec la protéase à sérine spécifique du site de lésion ; et
iii) identifier le peptide précurseur de liaison au fibrinogène candidat sous forme de peptide précurseur de liaison au fibrinogène si la formation de caillots ou l'agrégation plaquettaire est favorisée après la mise en contact du précurseur de liaison au fibrinogène candidat avec la protéase à sérine spécifique du site de lésion, mais une formation minimale de caillots ou une agrégation plaquettaire minimale est favorisée avant la mise en contact avec la protéase à sérine spécifique du site de lésion.
